# EUROPEAN PATENT APPLICATION

(11) **EP 0 540 927 A1**
(43) Date of publication of application: **12.05.1993**
(21) Application number: 92117827.3
(22) Date of filing: 19.10.1992
(51) Int. Cl.: A61B 17/34, A61B 19/00

(54) **Translucent cannula**

(30) Priority: 18.10.1991 US 781001
(71) Applicant: United States Surgical Corporation, Norwalk, Connecticut 06856 (US)
(72) Inventor: Shichman, Daniel, Trumbull, CT 06611 (US)
(74) Representative: Marsh, Roy David

(57) **Abstract**

Surgical devices derived from material having a level of absorbance, heat capacity, and thermal diffusivity such that when contacted with an incident or concentrated light at about 500-600nm, 1000-1100nm, and 10000-11000nm the material will not absorb a quantity of energy great enough to make it flash.

## Description

### TECHNICAL FIELD

The present invention relates to surgical instruments and more particularly to a trocar assembly.

### BACKGROUND OF THE INVENTION

Trocars are known in the art. In endoscopic procedures, a trocar is inserted into the body, such as through the abdominal wall, to perform minimally invasive surgery or diagnostic procedures. As a preliminary step, pneumoperitoneum is often created by inserting a needle, such as Verres needle, and introducing a gas to the peritoneal cavity to cause the abdominal wall to separate from the internal organs. The trocar generally consists of a cannula subassembly which includes a cannula housing having a central aperture aligned with a distally extending cannula. The trocar also includes an obturator subassembly consisting of an obturator housing having a distally extending obturator which may be secured to the obturator housing with a spring-biased protective tube. The obturator has a sharp tip and is configured and dimensioned to be received through the cannula housing aperture and the cannula. In the assembled position ready for use, the protective tube is able to slide proximally to expose the sharp tip of the obturator which protrudes from the distal end of the cannula. The trocar is inserted into the body the pressing the trocar against the patient's skin, causing the protruding obturator to make an incision in the skin so that the cannula can penetrate the body. Once the incision has been made the protective tube is able to slide distally under the force of the spring to again cover the sharp tip of the obturator, thereby shielding organs within the abdominal cavity from injury. After penetration, the obturator subassembly is removed from the cannula subassembly, leaving the cannula housing with the cannula extending into the peritoneal cavity. Trocars of this type are generally shown and described in U.S. Patent Nos. 4,601,710, 4,654,030, 4,902,280, 5,030,206 and 4,943,280. Although trocars are discussed herein primarily with reference to abdominal surgery, it will be understood that the present invention will find application to any use of a trocar, regardless of the surgical procedure or anatomical location.

Once the cannula is inserted into the peritoneal cavity, the surgeon may then perform endoscopic procedures using either conventional surgical instruments, such as graspers, dissectors, scissors, electrocautery instrumentation and lasers. Often a "YAG" laser which transmits light at about 1000nm to about 1100nm is the preferred laser because its beam can be transported by a fiber optic which can be threaded through the cannula to accurately and conveniently focus on its desired target. Other lasers such as the "CO₂" laser do not use a fiber optic and therefore must be focused and fired through the cannula from outside the body, which may result in a more time consuming and less accurate procedure.

Cannulas are presently made from materials such as metal, opaque plastics, and even fiberglass laminates. If inadvertently contacted by the laser beam during surgery, such materials will absorb so much of the energy from the beam that they may release a visible spark or flame (hereinafter referred to as flashing) and damage the fiberoptic. During laser surgery, smoking may occur from the burning of tissue. Clinically acceptable amounts of smoking are expected and acceptable. Furthermore, the gas injected into the abdominal cavity to create the pneumoperitoneum may be of a flammable nature. Therefore, flashing is clearly to be avoided.

The protection of surgeons, patients and equipment from direct or incident exposure to laser light with laser shields is known. These shields are designed to inhibit the progression of the light beam along its path by making it non-coherent. However, in endoscopic procedures creating a larger or separate incision to insert a protective shield is impractical at best.

### SUMMARY OF THE INVENTION

Therefore the present invention provides a cannula made from translucent material which can tolerate, without flashing, the inadvertent impact of the beam of commercially available surgical lasers, e.g. the 1000nm to 2100nm wavelength beam of a "YAG" laser, a 500nm to 550nm wavelength beam of a "YAG/KTP" laser and a 10,000nm to 11,000nm wavelength beam of a CO₂ laser.

In accordance with the present invention there is provided a cannula derived from a material selected such that the amount of energy absorbed by the material when exposed to light from commercially available laser devices, and more particularly of about 500-600nm, 1000-2000nm and 10,000-11,000nm wavelengths, for at least 0.2 seconds does not cause the material to spark or flame (hereinafter referred to as "flashing"). In one embodiment the cannula comprises about 1 to about 40 parts by weight of the cannula, of glass fibers and about 60 to about 99 parts by weight of the cannula of a polyamide. In another embodiment of the present invention, there is provided a cannula fabricated by insert molding where up to about 95% of the length of the cannula is made from materials that will flash the remainder made from materials not capable of flashing.

Other advantages of the present invention will be apparent from the following detailed description and the accompanying drawings.

### BRIEF DESCRIPTION OF DRAWINGS

Figure 1 illustrates a schematic view of a cannula and fiber optic inserted into a body cavity.

Figure 2 illustrates a cross sectional view of a trocar comprising a cannula of the present invention.

Figure 3 illustrates a side perspective view of a cannula of the present invention fabricated by insert molding.

### DETAILED DESCRIPTION OF THE INVENTION

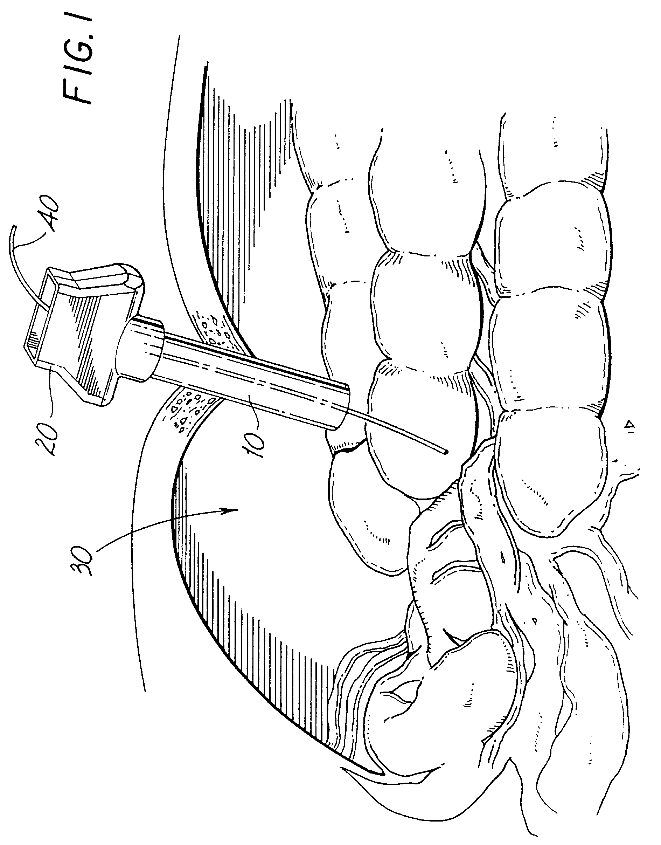

Referring now to the drawings:
A cannula as seen in Figure 1 is formed as a tubular body having a central axis and opposite proximal and distal open ends, which thus define a cannula passage in its interior. During surgery, the cannula 10 extends into a body cavity such as the peritoneal cavity 30 and a fiber optic is threaded through the cannula housing 20 which is connected to the proximal opening in the cannula. Generally, the cannula measures about 100mm to about 150mm in length and may be either rigid or flexible.

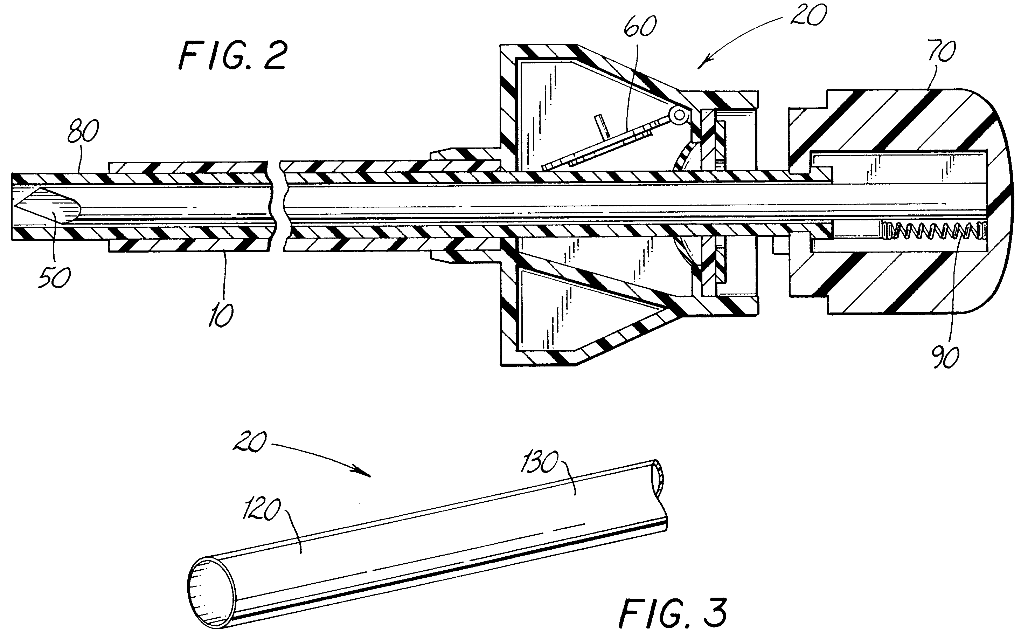

As seen in Figure 2, cannula 10 may be connected to cannula housing 20 having a gas sealing means 60. The cannula is configured and dimensioned so as to receive an obturator 50 or other endoscopic instrumentation such as a fiberoptic of a YAG laser 40 or the beam of CO₂ laser. The obturator may be connected to the obturator housing 70. A protective tube 80 optionally may be disposed concentrically around the obturator which may be biased outward from the obturator housing by biasing means 90. Such trocars and cannula are more fully described in U.S. Patents 5,030,206, 4,943,280, 4,902,280, 4,601,710 and 4,654,030 and co-pending U.S. Application Serial No. 07/593,676 incorporated herein by reference. The cannula of the present invention comprises about 1 to about 40 parts by weight of the cannula, of glass fibers and about 60 to 99 parts by weight of the cannula of a polyamide. As seen in Figure 3, cannula 20 may be fabricated by insert molding from materials capable of flashing 120 extending from its distal end up to about 95% of the length of the cannula, the remainder being fabricated from the non-flashable material 130 described herein.

Polyamides suitable for use in the preparation of the cannula of this invention may be made by any known method, including the polymerization of a monoamino-monocarboxylic acid or a lactam thereof having at lest 2 carbon atoms between the amino and carboxylic acid or a lactam thereof together with substantially equimolar proportions of a diamine and a dicarboxylic acid. (The term "substantially equimolar" proportions includes both strictly equimolar proportions and slight departures therefrom which are involved in conventional techniques for stabilizing the viscosity of the resultant polyamides.) The dicarboxylic acid may be used in the form of a functional derivative thereof, for example, an ester or acid chloride.

Examples of the aforementioned monoamino-monocarboxylic acids or lactams thereof which are useful in preparing the polyamides include those compounds containing from 2 to 16 carbon atoms forming a ring with the -CO-NH-group in the case of a lactam. Particular examples of aminocarboxylic acids and lactams are E-aminocaproic acid, butyrolactam, pivalolactam, E-caprolactam, capryllactam, enantholactam, undecanolactam, dodecanolactam and 3- and 4- aminobenzoic acids.

Diamines suitable for use in the preparation of the polyamides include the straight chain and branched chain alkyl, aryl and alkaryl diamines. Such diamines include, for example, those represented by the general formula

H₂N(CH₂)ₙNH₂

wherein n is an integer of from 2 to 16. Illustrative diamines are trimethylenediamine, tetramethylenediamine, pentamethylenediamine, octamethylenediamine, hexamethylenediamine (which is often preferred), trimethylenemethylenediamine, m-phenylenediamine and m-xyllylenediamine.

The dicarboxylic acids may be represented by the formula

HOOC-W-COOH

wherein W is a divalent aliphatic or aromatic group containing at least 2 carbon atoms. Examples of aliphatic acids are sebacic acid, suberic acid, glutaric acid, pimelic acid and adipic acid. Examples of aromatic acids are isophthalic and terephthalic acids.

Typical examples of the polyamides or nylons, as these are often called, include polyamide -6, 66, 11, 12, 63, 64, 6/10 and 6/12 as well as polyamides from terephthalic acid and/or isophthalic acid and trimethylhexamethylenediamine; from adipic acid and m-xylylenediamines; from adipic acid, azelaic acid and 2,2 -bis (p-aminocyclohexyl) propane and from terephthalic acid and 4,4'-diaminodicyclohexylmethane. Mixtures and/or copolymers of two or more of the foregoing polyamides or prepolymers thereof, respectively, are also suitable for use within the scope of the present invention. Preferred polyamides are polyamide -6, 66, 11 and 12, more preferably polyamide -6 or -66.

The cannula of the present invention may optionally comprise up to about 1 part by weight of the cannula of filler material. Suitable filler material includes dyes, pigments flow agents and binding agents.

The cannula may be molded in conventional molding apparatus. It is often preferably fabricated by injection molding by reason of the excellent properties of injection molded objects, and the availability of injection molding devices in commercial processing facilities.

It is believed that a material will flash provided its absorbance, heat capacity and thermal diffusivity are such that the material not only absorbs energy if hit by a beam from a laser source, but also that the amount of energy that said material is able to absorb increases until this energy reaches a level where flashing may occur.

In order that those skilled in the art may be better able to practice the present invention, the following examples are given as illustrations of the superior anti-flammability characteristics of the cannula of the present invention. It should be noted that the invention is not limited to the specific details embodied in the examples.

### Example 1

A cannula derived from a material comprising 33% glass fiber and 67% nylon 6,6 commercially available from E.I. Dupont and DeNemours Co. as Zytel GRZ having a thickness of about 0.75 mm was exposed in an ambient environment for 1 second to a 1060nm wavelength lightbeam from the fiberoptic of a YAG laser commercially available from Surgical Lasers Technology, Inc. The exposure occurred through contact with the fiberoptic. The cannula did not flash. The cannula was then exposed to a 530nm wavelength beam from a YAG laser fiberoptic (hereinafter called KTP). The cannula again did not flash although some smoke was present.

### Comparative Example 1

A cannula derived from a material comprising 40% carbon and 60% nylon 6,6 was exposed to the conditions of Example 1. Exposure to both the 1060nm and 530nm wavelengths caused the cannula to smoke and flash.

### Comparative Example 2

A cannula derived from a material comprising Stat Kon RCL 4033 commercially available from LNP Engineering Plastics Corporation was subjected to the conditions of Example 1. Exposure to the 1060nm wavelength caused smoke and exposure to the 530nm wavelength caused both smoke and flashing.

### Comparative Example 3

A cannula derived from 15% glass fibers, and 70% acetal, coated with 15% teflon was exposed to the conditions of Example 1. Exposure to both the 1060nm and the 530nm wavelengths caused both flame and smoke to appear.

### Comparative Example 4

A cannula derived from 70% Lexan commercially available from General Electric Co. and 30% carbon fiber was exposed to the conditions of Example 1. Exposure to both the 1060nm and the 530nm wavelengths cause flame and smoke.

**TABLE I**

| MATERIAL | LASER | OBSERVATIONS |
|---|---|---|
| Example 1 | YAG | no flashing |
| | KTP | some smoke |
| Comparative Example 1 | YAG | flashing smoke |
| | KTP | flashing |
| Comparative Example 2 | YAG | flashing smoke |
| | KTP | flashing |
| Comparative Example 3 | YAG | flashing smoke |
| | KTP | smoke |
| Comparative Example 4 | YAG | flashing smoke |
| | KTP | flashing |

TABLE I illustrates that superior anti-flammability characteristics of the cannula of the present invention when exposed to the YAG laser.

### Example 2

A cannula similar to that of Example 1 was exposed to a 10,600nm wavelength light beam from a 10 watt C₂ laser commercially available from Laser Engineering, Inc. at a focal length of 300mm for 0.2 second. No flashing, smoke or soot were observed.

### Comparative Example 5

A cannula derived from 100 parts fiberglass was exposed to the conditions of Example 2. A flashing but no soot was observed.

### Comparative Example 6

A cannula derived from Lexan commercially available from General Electric Co. was exposed to the conditions of Example 6. No flame was present but the beam burned a hole through the cannula creating much soot.

### Comparative Example 7

A cannula derived from polysulfone was exposed to the conditions of Example 6. Both flames and soot were present.

### Comparative Example 8

A cannula derived from Ektar commercially available from Eastman Kodak Inc. was exposed to the conditions of Example 6. The laser burned a hole through the cannula.

### Comparative Example 9

A cannula derived from Ultem commercially available from General Electric Co. was exposed to the conditions of Example 6. Flames and soot were observed.

**TABLE II**

| MATERIAL | LASER | OBSERVATIONS |
|---|---|---|
| Example 2 | CO₂ | no flame |
| | | no soot |
| Comparative Example 5 | CO₂ | flame |
| Comparative Example 6 | CO₂ | burned hole through cannula and soot |
| Comparative Example 7 | CO₂ | flame and soot |
| Comparative Example 8 | CO₂ | burned hole through cannula |
| Comparative Example 9 | CO₂ | flame and soot |

TABLE II illustrates that superior anti-flammability characteristics of the cannula of the present invention when exposed to CO₂ laser.

Obviously, other modifications and variations of the present invention are possible in light of the above teachings. It is therefore to be understood that changes may be made in particular embodiments of the invention described which are within the full intended scope of the invention as defined by the claims. It is to be further understood that all patents mentioned above are to be incorporated herein by reference as well as that all parts are by weight of the total weight of the cannula.

## Claims

1. A cannula fabricated from a material selected such that the amount of energy absorbed by said material when exposed to a laser energy source for at least 0.2 seconds does not cause said material to flash.

2. A cannula according to claim 1 comprising
a. about 1 to about 40 parts by weight based on the total weight of the cannula of glass fibers; and
b. about 60 to about 99 parts by weight based on the total weight of the cannula, of a polyamide.

3. A cannula according to claim 2 wherein the polyamide is a polyamide -6 or a polyamide -66.

4. A cannula according to claim 1 which contains up to about 1.0% of filler material.

5. A cannula according to claim 4 wherein the filler material is selected from the group consisting of pigments, dyes, flow agents and binding agents.

6. A cannula according to claim 1 comprising about 33 parts by weight, based on the total weight of the cannula of the glass fibers.

7. A cannula according to claim 1 further comprising flashable material extending up to about 95% of the length of the cannula from its distal end.

8. In combination
a) a cannula housing; and
b) a cannula fixedly secured to the cannula housing, wherein the cannula is fabricated from a material selected such that the amount of energy absorbed by said material when exposed to a laser energy source for at least 0.2 seconds does not cause said material to flash.

9. The combination according to claim 8 wherein the cannula comprises
a) about 1 to about 40 parts by weight based on the total weight of the cannula of glass fibers; and
b) about 60 to about 99 parts by weight based on the total weight of the cannula, of a polyamide.

10. The combination according to claim 9 wherein the polyamide is a polyamide -6 or a polyamide -66.

11. The combination according to claim 8 wherein the cannula contains up to about part by weight of the cannula, of filler material.

12. The combination according to claim 11 wherein the filler material is selected from the group containing pigments, dyes banding agents and flow agents.

13. The combination according to claim 9 wherein the cannula comprises about 33 parts, by weight of the cannula, of the glass fibers.

14. The combination according to claim 8 wherein the cannula defines a tubular passage configured and dimensioned to receive endoscopic instrumentation means.

15. The combination according to claim 8 wherein the cannula housing includes gas sealing means adapted to cooperate with endoscopic instrumentation inserted therethrough.

16. The combination according to claim 8 wherein the cannula is rigid.

17. The combination according to claim 8 wherein the cannula is flexible.

18. The combination according to claim 8 wherein the cannula is configured and dimensioned to extend through a body wall and into a body cavity.

19. The combination according to claim 8 wherein the cannula measures about 100mm to about 150mm in length.

20. The combination according to claim 8 wherein the cannula further comprises non-flashable material extending from the distal end of the cannula up to about 95% of the length of the cannula.

21. In combination
a) a trocar assembly comprising:
an elongated obturator having a sharp tip and an obturator housing;
b) a cannula housing; and
c) a cannula, fixedly secured to the cannula housing, wherein the cannula is fabricated from a material selected such that the amount of energy absorbed by said material when exposed to a laser energy source for at least 0.2 seconds does not cause said material to flash.

22. The combination according to claim 21 wherein the cannula comprises:
a) about 1 to about 40 parts by weight based on the total weight of the cannula, of glass fibers; and
b) about 60 to about 99 parts by weight based on the total weight of the cannula, of a polyamide.

23. The combination according to claim 21 wherein the polyamide is a polyamide -6 or a polyamide -66.

24. The combination according to claim 22 wherein the cannula contains up to about 1 part by weight of the cannula, of filler material.

25. The combination according to claim 24 wherein the filler material is selected from the group containing pigments, dyes binding agents and flow agents.

26. The combination according to claim 23 wherein the cannula comprises about 33 parts, by weight of the cannula, of the glass fibers.

27. The combination according to claim 21 wherein the cannula defines a tubular passage configured and dimensioned to receive endoscopic instrumentation means.

28. The combination according to claim 21 wherein the cannula housing includes gas sealing means adopted to cooperate with endoscopic instrumentation inserted there through.

29. The combination according to claim 21 wherein the cannula is rigid.

30. The combination according to claim 21 wherein the cannula is flexible.

31. The combination according to claim 21 wherein the cannula is configured and dimensioned to extend through a body wall and into a body cavity.

32. The combination according to claim 21 wherein the cannula measures about 100mm to about 150mm.

33. The combination according to claim 21 wherein the cannula further comprises non-flashable material extending from the distal end of the cannula up to about 95% of the length of the cannula.
